(19) Europäisches Patentamt
European Patent Office
Office européen des brevets

(11) Veröffentlichungsnummer: **0 065 595**
**B1**

(12) # EUROPÄISCHE PATENTSCHRIFT

(45) Veröffentlichungstag der Patentschrift: **26.02.86**

(51) Int. Cl.⁴: **C 07 D 251/48**, C 09 B 62/08, C 09 B 44/02, D 06 P 1/38, D 21 H 3/80

(21) Anmeldenummer: **81106799.0**

(22) Anmeldetag: **01.09.81**

(54) **Wasserlösliche Triazinverbindungen, ihre Herstellung und ihre Verwendung.**

(30) Priorität: **08.04.81 DE 3114088**

(43) Veröffentlichungstag der Anmeldung: **01.12.82 Patentblatt 82/48**

(45) Bekanntmachung des Hinweises auf die Patenterteilung: **26.02.86 Patentblatt 86/9**

(84) Benannte Vertragsstaaten: **CH DE FR GB IT LI NL SE**

(56) Entgegenhaltungen:
**DE - A - 1 419 859**
**FR - A - 2 262 512**

(73) Patentinhaber: **BAYER AG, Konzernverwaltung RP Patentabteilung, D-5090 Leverkusen 1 Bayerwerk (DE)**

(72) Erfinder: **Stöhr, Frank-Michael, Dr., Höhenstrasse 130, D-5090 Leverkusen 3 (DE)**
Erfinder: **Nickel, Horst, Dr., Fontanestrasse 23, D-5090 Leverkusen 1 (DE)**

**EP 0 065 595 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung sind wasserlösliche Triazinverbindungen, die in ihrer Betainform der Formel

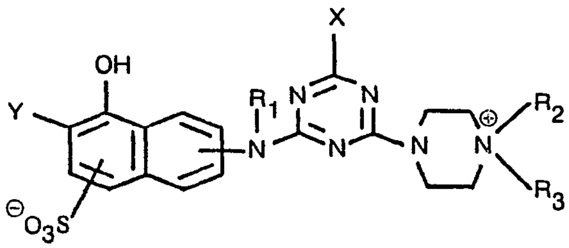

(I)

entsprechen, worin

$R_1$ Wasserstoff oder Methyl,

$R_2$ und $R_3$ unabhängig voneinader Wasserstoff, $C_1$—$C_4$-Alkyl, $C_3$- oder $C_4$-Alkenyl, Benzyl, Phenylethyl, die durch Hydroxy, $C_1$- bis $C_4$-Alkoxy, Halogen, Cyan, Amino oder Mono- und Di-$C_1$—$C_4$-alkylamino oder deren Ammoniumverbindungen oder Tri-$C_1$—$C_4$-alkylammonium und der Benzyl- und Phenylethylrest zusätzlich durch $C_1$—$C_4$-Alkyl substituiert sein können,

X Halogen, Hydroxy, $C_1$—$C_4$-Alkoxy, $C_1$—$C_4$-Alkyl, Phenyl oder einen Rest der Formel

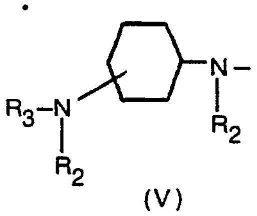

(V)

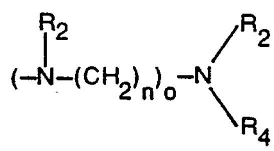

(VI)

oder

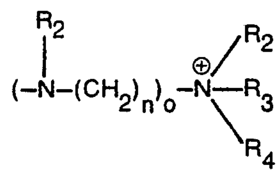

(VII)

n 2 oder 3 und zusätzlich 0, wenn o = 1 ist,

o 0, 1 oder 2,

$A^{\ominus}$ ein Anion,

$R_4$ den Rest $R_2$ und zusätzlich gegebenenfalls durch Halogen, $C_1$—$C_4$-Alkyl, Hydroxy, $C_1$—$C_4$-Alkoxy und/oder den Rest

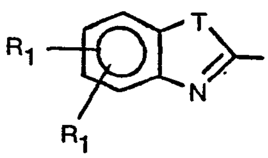

T = S, NH

(VIII)

substituiertes Phenyl oder Naphthyl, Benzthiazolyl (2) oder Isobenzthiazolyl (3) oder

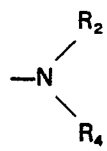

einen Pyrrolidin-, Piperidin- oder Morpholinring oder einen Ring der Formel

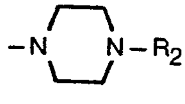

oder

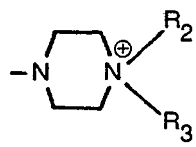

$A^{\ominus}$

Y Wasserstoff oder den Rest D-(N=N-A)$_z$—N=N-,

D den Rest einer Diazokomponente der Benzol-, Naphthalin- oder heterocyclischen Reihe,

A den Rest einer Kupplungskomponente der Benzol- oder Naphthalinreihe und

m und z 0 oder 1 bedeuten, und worin

die Summe der basischen und kationischen Gruppen größer ist als die Anzahl der Sulfonsäuregruppen, ihre Herstellung und — wenn Y für den Rest D—(N=N—A)$_z$—N=N— steht — h Verwendung zum Färben von synthetischen und natürlichen Materialien, insbesondere Papier, und — wenn Y für Wasserstoff steht — ihre Verwendung zur Herstellung von Azofarbstoffen.

Bevorzugt sind Verbindungen der Formel (I), worin die Sulfonsäuregruppe in 3-Stellung und $R_1$ für Wasserstoff stehen.

Hervorzuheben sind wasserlösliche, kationische Azofarbstoffe der Formel

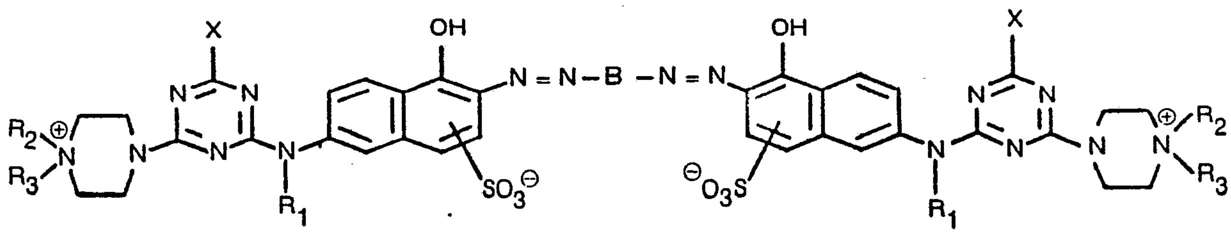

(II)

worin

B den Rest einer aromatischen Tetrazokomponente darstellt,

X, $R_1$, $R_2$ und $R_3$ die Bedeutung der Formel (I) haben, und worin die Summe der basischen und kationischen Gruppen größer als die Anzahl der Sulfonsäuregruppen ist.

Die Anzahl der basischen und kationischen Gruppen ist von entscheidender Bedeutung für die Wasserlöslichkeit der Farbstoffe, da die Sulfonsäuregruppe mit der basischen oder kationischen Gruppe ein inneres, schwerlösliches Salz bilden kann und erst überzählige basische Gruppen in Form der Säureadditionssalze und/oder kationische Gruppen die Wasserlöslichkeit bewirken.

Dabei kann (können) die zusätzliche(n) basische(n) und/oder kationische(n) Gruppe(n) sowohl in A, D und B als auch in X lokalisiert sein.

Halogen steht insbesondere für Fluor, Chlor oder Brom.

Von den Farbstoffen der Formeln (I) und (II) sind die der Formeln

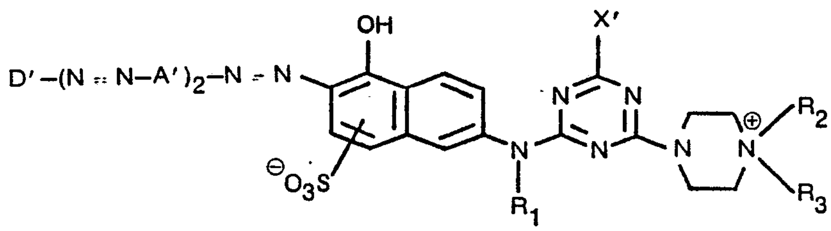

(III)

und

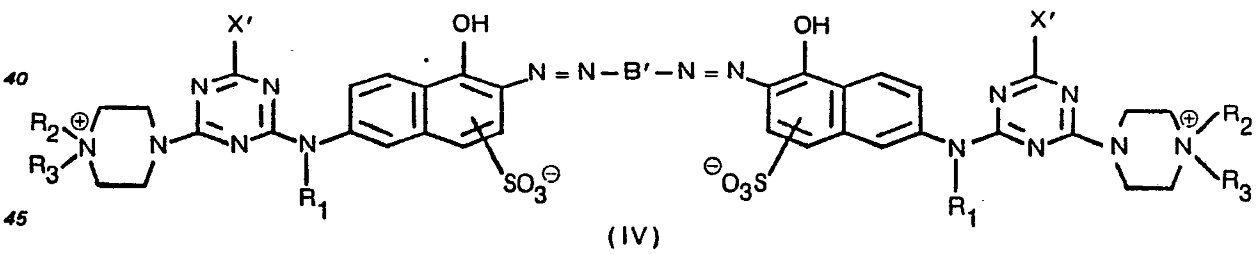

(IV)

bevorzugt, worin

X' einen Rest der Formel (V), (VI) oder (VII),

D' einen Phenyl-, Naphthyl-, Benzthiazolyl- oder Dibenzofuranylrest,

A' einen Phenylen- oder Naphthylenrest,

B' einen Phenylen- oder Naphthylenrest oder einen Rest der Formel

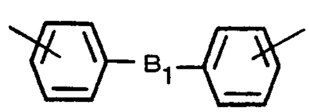

bedeuten, worin

$B_1$ eine direkte Bindung, $-(CH_2)_p-$, $-NH-CO-$, $-O-$, $-SO_2-$, $-NHCONH-$, $-O-(CH_2)_p-O-$, $-NH-CO-(CH_2)_p-CO-NH-$, $-CO-NH-(CH_2)_p-NH-CO-$ oder

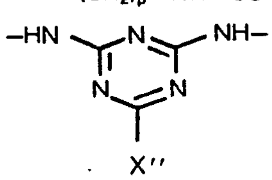

p 2 oder 3 bedeuten,

D', A' und die isocyclischen Ringe von B' durch Halogen, $C_1$—$C_4$-Alkyl, $C_1$—$C_4$-Alkoxy, Acetylamino, Benzoylamino, Phenoxy, die Gruppen (VI), (VII),

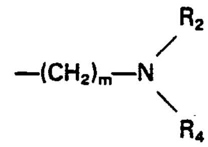
,
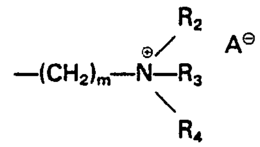

und/oder eine Sulfonsäuregruppe substituiert sein können, und

D' und A' außerdem durch (VIII) oder

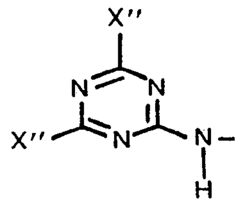

substituiert sein können, worin

X'' den Rest X' und die Reste

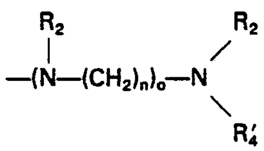
oder
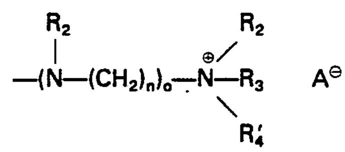

bedeutet, worin

$R_4'$ gegebenenfalls durch die Reste

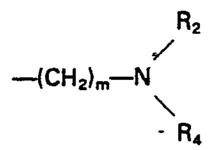
oder
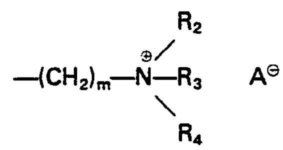

substituiertes Phenyl oder Naphthyl bedeutet, und

m und z 0 oder 1 sein können.

Die Auswahl der Diazo- bzw. Tetrazokomponenten muß in der Weise erfolgen, daß die Bedingung der Wasserlöslichkeit erfüllt wird, d.h., daß das entstehende Farbmolekül mindestens eine basische oder kationische Gruppe mehr enthält, als das Molekül Sulfonsäuregruppen besitzt.

Besitzt also die Kupplungskomponente (I) (Y = Wasserstoff) neben der basischen oder kationischen Gruppe in X eine zweite basische oder kationische Gruppe, so kann eine beliebige aromatische, carbocyclische oder heterocyclische Diazo- bzw. Tetrazokomponente — die frei ist von anionischen Gruppen — wie beispielsweise Anilin, Aminoazobenzol, Aminonaphthalin, 4,4'-Diamino-benzoyl-anilid, 4,4'-Diamino-3,3'-dimethyl- bzw. -dimethoxydiphenyl, 4,4'-Diaminodiphenyl-ethan (1,2) Verwendung finden.

Besitzt die Kupplungskomponente (I) (Y = Wasserstoff) dagegen nur eine basische oder kationische Gruppe, so muß die aromatische carbocyclische oder heterocyclische Diazo- bzw. Tetrazokomponente mindestens eine basiche oder kationische Gruppe aufweisen, um in der Salzform der basischen Gruppe oder der kationischen Gruppe die Wasserlöslichkeit zu erreichen.

Geeignete Diazokomponenten dieser Art sind beispielsweise Anilin-3- oder -4-trimethylammonium-chlorid, -methosulfat, -sulfat, -benzolsulfonat, -tosylat, 3- oder 4-Aminobenzyldi- oder -trimethylammoniumchlorid, -methosulfat oder 2-Aminonaphthalin-5-methylentrimethylammoniummethosulfat.

Hierzu zählen auch Aminoazoverbindungen mit gegebenenfalls über eine Methylengruppe gebundenen, gegebenenfalls alkylierten Amino- oder Ammoniumgruppen, wie beispielsweise

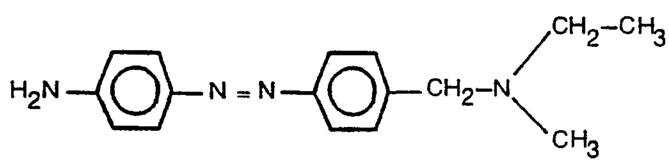

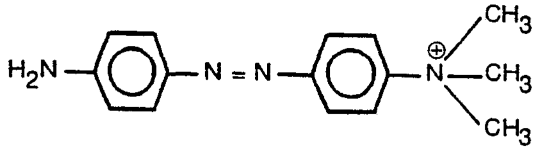
$Cl^{\ominus}$ bzw. $CH_3SO_4^{\ominus}$

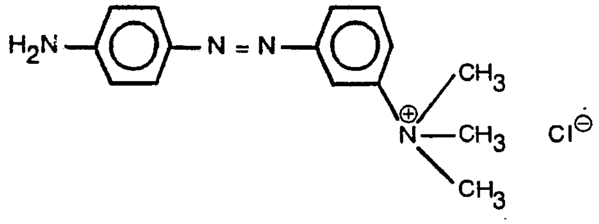

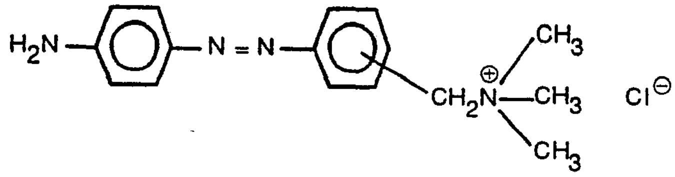

ferner Verbindungen der oben genannten Art, bei denen der Benzolkern durch Methyl, Methoxy, Ethoxy oder Chlor noch weiter substituiert ist, sowie die Kupplungsprodukte von 2-Aminonaphthalin-5-methylen-trimethyl-ammoniumchlorid → Anilin bzw. → 3-Methylanilin bzw. → 2-Methoxyanilin bzw. → 2-Methoxy-5-methylanilin.

Als Anion$^{\ominus}$ kommen übliche farblose organische und anorganische Anionen, beispielsweise Chlorid, Bromid, Jodid, Hydroxyl, Hydrogensulfat, Sulfat, Nitrat, Dihydrogenphosphat, Hydrogenphosphat, Phosphat, Carbonat, Methosulfat, Ethosulfat, Formiat, Acetat, Propionat, Benzolsulfonat und Toluolsulfonat in Betracht.

Das Anion ist im allgemeinen durch das Herstellungsverfahren gegeben. Die Anionen können in bekannter Weise gegen andere Anionen ausgetauscht werden. Vorzugsweise liegen die Chloride, Hydrogensulfate, Sulfate, Methosulfate, Phosphate, Formiate oder Acetate vor.

Die Kupplungskomponenten (I) (Y = H) werden in bekannter Weise im allgemeinen folgendermaßen hergestellt: Die Naphthalin-sulfonsäure (IX), vorzugsweise die 3-Sulfonsäure, wird zunächst mit Cyanurhalogenid, bevorzugt -chlorid oder -fluorid, zur 1:1-Verbindung umgesetzt. Anschließend wird durch Halogenaustausch, beispielsweise mit Ammoniak oder Aminen HX der X-Substituent eingeführt.

Nach Einführung des Substituenten X in der zweiten Stufe (bei 20—50°C) wird in dritter Stufe bei 70—95°C die Kondensation mit der Aminoverbindung

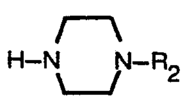

oder

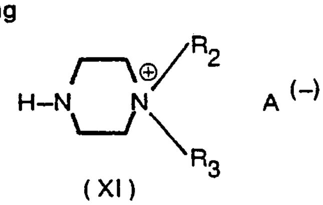

(X) (XI)

vorgenommen.

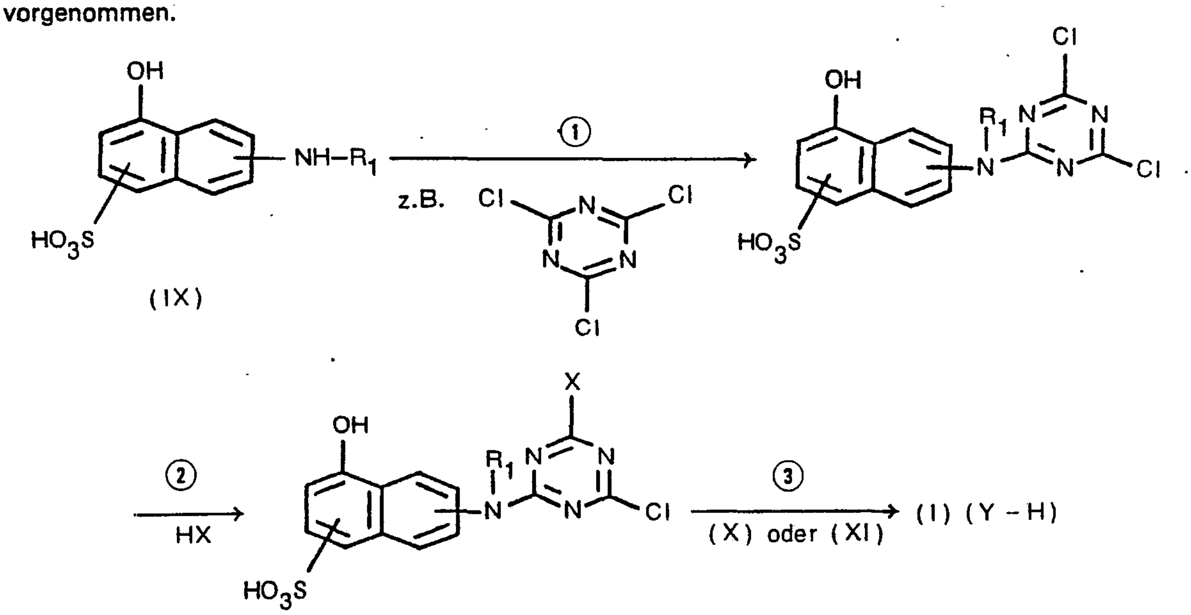


(IX)

Falls X = F, Cl, Br wird die Reaktion mit (X) oder (XI) in zweiter Stufe durchgeführt.

Selbstverständlich können die einzelnen Kondensationsschritte vertauscht werden und beispielsweise zuerst die Verbindungen (X) oder (XI) mit dem Cyanurhalogenid kondensiert werden.

Bevorzugte Verbindungen (X) sind unsubstituiertes und N-Methyl- und N-(2-Hydroxyethyl)-piperazin sowie besonders N-(2-Aminoethyl)-piperazin.

Als Amine HX seien folgende genannt: $C_1$—$C_4$-Mono- bzw. Dialkylamine wie Methyl-, Ethyl-, Chlorethyl-, n-Propyl-, i-Propyl-, Butyl-, Hydroxy-ethyl-, 2-Methoxy-ethyl-, Dimethyl-, Diethyl-, Di-i-propyl-, Di-n-propyl-, Diethanol-, Di-iso-propanol-, Di-iso-butyl-, Methyl-ethyl-, Methyl-ethanol- und Ethylethanolamin, 3-Methylaminopropionitril, Cycloalkylamine wie Pyrrolidin, Piperidin, Morpholin, Piperazin, N-Methylpiperazin, N-(2-Hydroxyethyl)-piperazin, N-(2-Aminoethyl)-piperazin oder entsprechende Piperazinium-Salze; Aralkylamine wie Benzylamin, Benzyl-methylamin, Benzylethanolamin, die im Phenylkern durch Chlor oder Methyl substituiert sein können, Diamine wie Ethylendiamin, N,N'-Dimethylethylendiamin, 1-Amino-2-diethylaminoethan, Propylendiamin, N-Methyl- und N.N-Dimethyl- oder -ethylpropylendiamin, Dipropylentriamin, Diethylentriamin, N,N',N''-Trimethyldiethylen-triamin, 1,4-Diaminocyclohexan, aromatische Amine wie Anilin, N-Methyl oder Ethyl- oder Hydroxyethylanilin, die im Phenylkern in o-, m- oder p-Stellung durch Methyl, Ethyl, Chlor, Methoxy oder Ethoxy substituiert sein können, 1-Naphthylamin, Dehydrothiotoluidin oder -xylidin, 2-Aminobenzothiazol, 3-Aminoisobenzothiazol, ferner Methyl- und Dimethylhydrazin.

Als Amine HX seien zusätzlich folgende genannt: Aromatische Amine wie Anilin, N-Methyl- oder Ethyl- oder Hydroxyethylanilin, die im Phenylkern in m- oder p-Stellung durch N,N-Dimethyl- oder N,N-Diethylamino, Trimethylammoniumchlorid, -methosulfat, -acetat oder -tosylat, Methylendimethyl-, -diethylamino oder Methylentrimethylammoniumchlorid substituiert sind, oder 2-Aminonaphthalin-5-methyltrimethylammoniumchlorid.

Die neuen Azofarbstoffe (I) (Y = D—(N=N—A)$_z$—N=N—) werden nach üblichen, in der Azochemie bekannten Verfahren hergestellt (siehe z.B. Houben Weyl: Methoden der organischen Chemie, Band X/3, Georg Thieme-Verlag, Stuttgart, 1965 ab Seite 226 und Seite 270) durch Kupplung von Diazonium- bzw. Tetrazoniumverbindungen mit Kupplungskomponenten der Formel (I) (Y = H), vorzugsweise in wäßrigem Medium. Falls X = Alkyl oder Aryl, sind natürlich nur 2 austauschbare Halogen-Substituenten im Triazinring zur Verfügung, wobei wahlweise zuerst (X) bzw. (XI) oder die Aminonaphtholsulfonsäure (IX) kondensiert werden kann.

Die Farbstoffe werden zum Färben von mit kationischen Farbstoffen färbbaren Materialien verwendet. Genannt seien beispielsweise; Polyacrylnitril, sauer modifizierte Polyester, z.B. Polyglykolterephthalate, wie sie in der belgischen Patentschrift 549 179 oder US-Patentschrift 2 893 816, beschrieben werden, sauer modifizierte Polyamide, tannierte vegitabilische Fasern (Baumwolle), Leder und vorzugsweise Papier, Geeignet sind die Farbstoffe zum Färben von geleimtem sowie ungeleimtem Papier, wobei von gebleichtem oder ungebleichtem Zellstoff ausgegangen werden kann und Laub- oder Nadelholz-Zellstoff wie Birken- und/oder Kiefernsulfid und/oder Sulfat-Zellstoff verwendet werden kann.

Die Farbstoffe werden sowohl als Pulver- bzw. Granulat-Präparationen als auch in Form von konzentrierten Lösungen zum Einsatz gebracht. Pulver-Präparationen werden in üblicher Weise mit Stellmaterialien wie Natriumsulfat, -phosphat, -chlorid, -acetat in Gegenwart von Entstaubungsmitteln eingestellt oder die Farbstoffe werden direkt als Sprühtrocknungspräparationen in den Handel gebracht. Konzentrierte Farbstofflösungen können wäßriger oder wäßrig/organischer Art sein, wobei übliche, umweltfreundliche und möglichst gut abbaubare Zusätze bevorzugt werden, wie anorganische oder organische Säuren, vorzugsweise Essigsäure, Hydroxyessigsäure, Ameisensäure, Milchsäure, Zitronensäure, Methansulfonsäure, Phosphorsäure, Amide wie Formamid, Dimethylformamid, Harnstoff, Alkohole wie Glykol, Diglykol, Diglykolether, vorzugsweise Methyl-, Ethyl- oder Butylether.

Die Farbstoffe besitzen ein ausgezeichnetes Ziehvermögen und sehr gute Allgemein-Echtheiten. Papierfärbungen zeichnen sich durch sehr gute Naßechtheiten sowie Alaun-, Säure- und Alkali-Echtheit aus. Sie besitzen eine überraschend hohe Lichtechtheit, bei gleichzeitig hoher Klarheit und Farbstärke.

Aus der DE—A 29 15 323 sind Azofarbstoffe bekannt, deren Summe von kationischen und basischen Gruppen ebenfalls größer ist als die Anzahl der Sulfonsäuregruppen, die aber im Gegensatz zu den neuen Farbstoffen der vorliegenden Erfindung keinen Piperazinring tragen.

Beispiel 1

Je 125 g Eis und Wasser werden mit 92 g (0,5 Mol)Cyanurchlorid und 0,5 g Emulgator L5 zu einer feinen Dispersion verrührt. Danach tropft man bei 0—5°C während 3 Stunden 110 g (1,1 Mol) N-Methylpiperazin ein. Man läßt 1 Stunde bei 40—45°C nachrühren und gibt 0,39 Mol I-Säure hinzu. Man heizt 3 Stunden auf 95°C auf. Nach beendeter Kondensation liegt die Kupplungskomponente der Formel

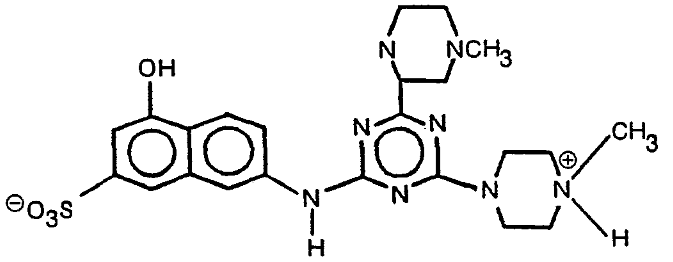

1.1

vor, die durch Ansäuern mit 28 %iger HCl auf pH 2 gestellt wird und als Lösung direkt zur Kupplung weiterverarbeitet werden kann.

Verwendet man die I-Säure als erste Kondensationskomponente und kondensiert in der 1. Stufe wie oben bei 0—5°C mit Cyanurchlorid, dann führt man zweckmäßig nach der 2. Stufe bei 40—50°C mit Anilin, N-Methylanilin, 2-Methylanilin, Methylamin, Dimethylamin, N,N-Dimethylpropylendiamin oder anderen der angeführten Amine HX eine Zwischenisolierung durch und kondensiert schließlich in der 3. Stufe bei 80—95°C mit β-Hydroxyethylpiperazin z.B. zur Kupplungskomponente

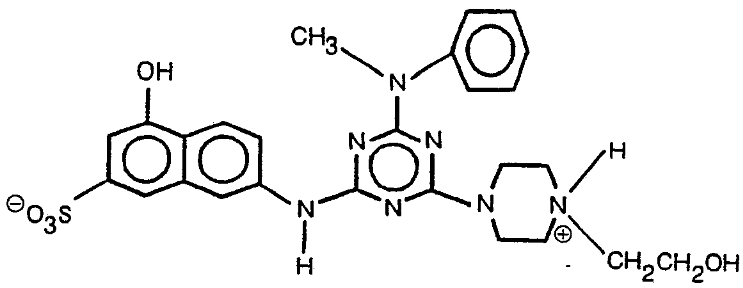
1.2

Kondensiert man in dritter Stufe mit einem Überschuß an Piperazin, so erhält man die Kupplungskomponente

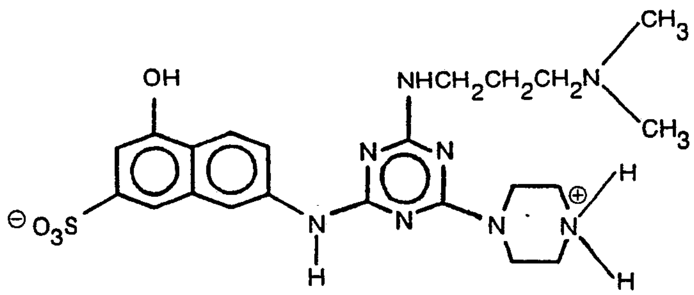
1.3

sofern die 2. Stufe mit N,N-Dimethylpropylendiamin durchgeführt wurde.

In der folgenden Tabelle sind weitere Kupplungskomponenten in ihrer Betainform angeführt:

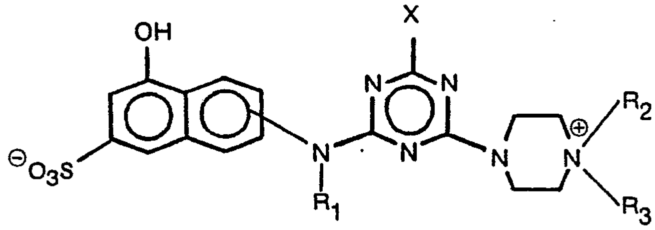

| | Säure | HX | $R_1$ | $R_2$ | $R_3$ |
|---|---|---|---|---|---|
| 1.4 | I-Säure | Anilin | H | $-CH_2CH_2OH$ | H |
| 1.5 | „ | Methylamin | „ | „ | „ |
| 1.6 | „ | Dimethylamin | „ | „ | „ |
| 1.7 | „ | Diethanolamin | „ | „ | „ |
| 1.8 | „ | Diisopropanolamin | „ | „ | „ |
| 1.9 | „ | Morpholin | „ | „ | „ |
| 1.10 | „ | Piperidin | „ | „ | „ |
| 1.11 | „ | Ammoniak | „ | „ | „ |
| 1.12 | „ | Ethylendiamin | „ | „ | „ |
| 1.13 | „ | 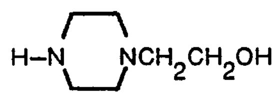 | „ | „ | „ |
| 1.14 | I-Säure | Dimethylamin | „ | $-CH_3$ | „ |
| 1.15 | „ | Diethanolamin | „ | „ | „ |
| 1.16 | „ | „ | „ | H | „ |
| 1.17 | „ | 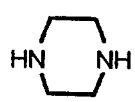 | „ | „ | „ |
| 1.18 | „ | Dimethylamin | „ | $-CH_3$ | $-CH_3$ |
| 1.19 | „ | 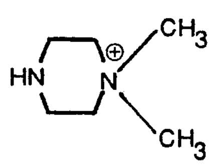 | „ | $-CH_3$ | $-CH_3$ |
| 1.20 | „ | Dimethylpropylendiamin | $-CH_3$ | $-CH_2CH_2OH$ | H |
| 1.21 | Gamma-Säure | „ | H | „ | „ |
| 1.22 | I-Säure | 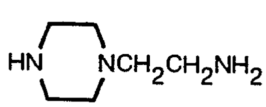 | „ | $-CH_2CH_2NH_2$ | „ |
| 1.23 | „ | Diethanolamin | „ | „ | „ |
| 1.24 | „ | Morpholin | „ | „ | „ |
| 1.25 | „ | Dimethylpropylendiamin | „ | „ | „ |
| 1.26 | „ | 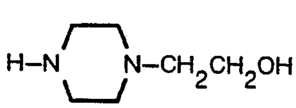 | „ | „ | „ |

Zur Herstellung der Kupplungskomponenten 1.18 und 1.19 kann man erst mit Methylpiperazin umsetzen und anschließend mit den üblichen Quaternierungsmitteln quaternieren. Ferner ist es ebenfalls möglich, erst nach der Kupplung die Quaternierung durchzuführen.

Beispiel 2

8,9 g (0,07 Mol) 4-Methoxyanilin werden in 200 ml Wasser mit 20 ml 28 %iger HCl gelöst und mit 50 ml 10 %iger $NaNO_2$-Lösung bei 0—5°C diazotiert. 43,7 g (0,076 Mol) Kupplungskomponente der Formel

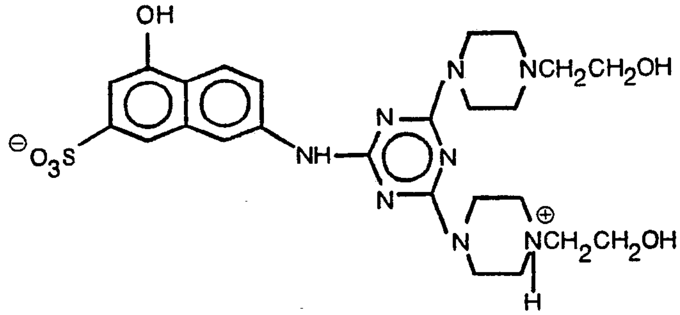

1.13

werden in 200 ml Wasser bei pH 3 gelöst. Man gibt die Diazotierung dazu und kuppelt bei 5—10°C. Der pH wird mit festem Natriumhydrogencarbonat auf 4,5—5 gehalten. Nach 3 Stunden wird mit 40 %iger Natronlauge auf pH 10 gestellt und der ausgefallene Farbstoff abgesaugt. Die Paste wird in 240 g 85 %iger $H_3PO_4$, 5 g Eisessig und 5 g Milchsäure bei 50°C gelöst und geklärt. Man erhält eine konzentrierte, gebrauchsfertige und kältestabile Lösung, die Papier in roten Tönen anfärbt.

Ersetzt man das 4-Methoxyanilin durch die folgenden Diazokomponenten, erhält man ebenfalls wertvolle Farbstoffe, die Papier in gelborangen bis blaustichig roten Tönen anfärben: 2-Methoxyanilin, 1-Amino-4-benzoylaminobenzol, 1-Amino-4-acetylaminobenzol, 2- und 4-Aminodiphenylether, 2,5-Dimethoxyanilin, 2,5-Dimethylanilin, 2-Methoxy-5-methylanilin, Anilin, 3-Chlor-4-methylanilin, 2-Aminonaphthalin-5-sulfonsäureamid, 4-Methylanilin, 4-Aminobenzoesäureanilid, 2-(4'-Aminophenyl)-6-methylbenzthiazol, 2-Aminodibenzofuran, 4-Chloranilin, 2,4- oder 2,5-Dichloranilin, 4-Ethoxyanilin, 2-Aminonaphthalin-5-methylentrimethylammoniumchlorid, 2-Aminonaphthalin-1-sulfonsäure-5-methylentrimethylammoniumchlorid, Anilin-3- oder 4-trimethylammoniumchlorid, 4-Aminoazobenzol, 2-Methyl-4-aminoazobenzol, 4-Aminoazobenzol-4'-trimethylammoniumchlorid, 4-Aminoazobenzol-4'-methylen-trimethylammoniumchlorid, 2-Methyl-4-aminobenzol-azo-2-naphthalin-5-methylentrimethylammoniumchlorid, 2-(4'-Amino-3'-methylphenyl)-4,6-dimethylbenzthiazol, 2-(4'-Aminophenyl)benztriazol, 2-(4'-Aminophenyl)-5-methylbenzimidazol, 2-(4'-Aminophenyl)-4-methylbenzimidazol,

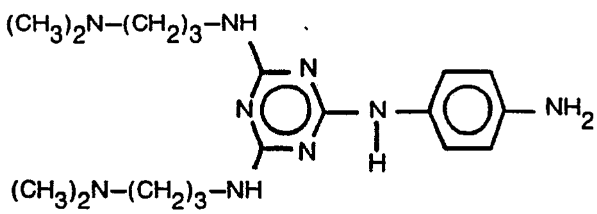

Verwendet man als Diazokomponente die Bis-diazokomponenten

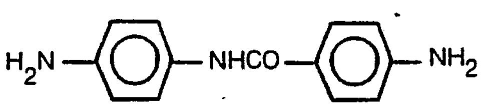 , 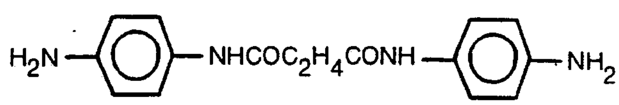 ,

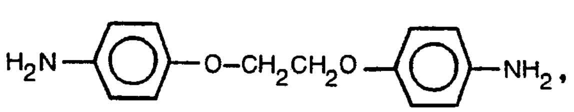 , 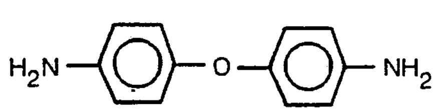 ,

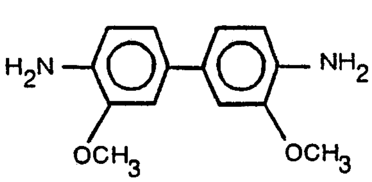 , 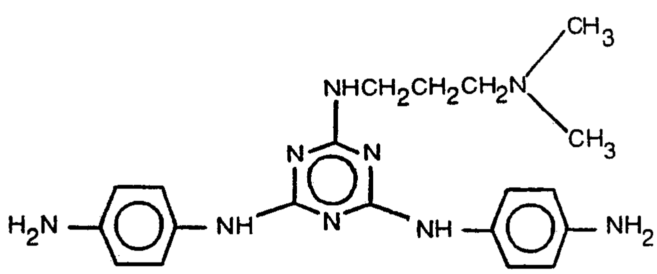

so erhält man ebenfalls rote bis violette Farbstoffe.

Unter Verwendung der Kupplungskomponenten 1.1, 1.3, 1.10, 1.12, 1.17, 1.19, 1.20 bis 1.26 werden bei Einsatz der obigen Diazokomponenten in den Farbtönen ähnliche Farbstoffe erhalten wie mit der Kupplungskomponente 1.13.

Beispiel 3

Die salzsaure Lösung von 25 Teilen (0,1 Mol) 2-Aminonaphthalin-5-methylentrimethylammoniumchlorid wird in üblicher Weise bei 0—5°C mit 70 ml 10 %iger $NaNO_2$-Lösung diazotiert.

75 Teile (0,11 Mol) der Kupplungskomponente der Formel

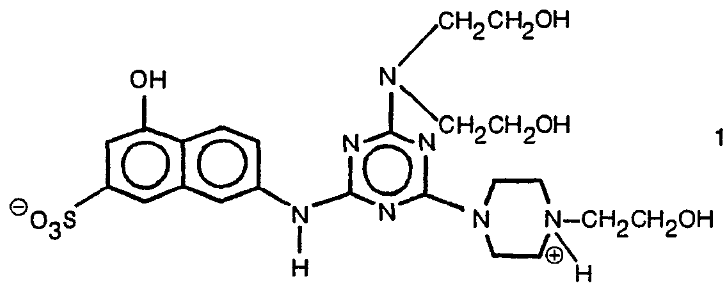

1.7

werden in 400 ml Wasser mit 40 %iger NaOH gelöst und anschließend mit HCl auf pH 5 gestellt, wobei die Verbindung ausfällt. Man gibt die Diazotierung dazu und kuppelt bei 5—10°C mit fester $NaHCO_3$ und pH 5,5—6. Nach 3 Stunden wird der ausgefallene Farbstoff abgesaugt. Die Paste wird in 400 ml $H_2O$ angeschlagen und mit 40 %iger NaOH auf pH 13 gestellt. Der Farbstoff wird isoliert und mit der gleichen Menge Ameisensäure bei Raumtemperatur gelöst und geklärt. Man erhält eine konzentrierte und kältestabile gebrauchsfertige Lösung, die Papier in scharlachroten Tönen färbt.

Bei Verwendung von Anilin-3- oder -4-trimethylammoniumchlorid oder Anilin-3- oder -4-methylentrimethylammoniumchlorid werden orangefarbene Farbstoffe erhalten.

4-Amino-azobenzol-4'-trimethylammoniumchlorid als Diazokomponente ergibt einen blaustichigroten Farbstoff.

Bei Einsatz von

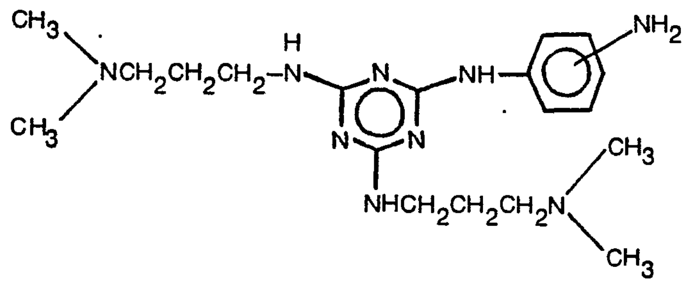

erhält man bei dem meta-Produkt ein scharlach und bei dem para-Produkt ein blaustichiges Rot.

Setzt man als Kupplungskomponente anstelle der Verbindungen 1.7 die Kupplungskomponenten 1.1—1.6 und 1.8—1.26 ein, so erhält man mit dem obigen Diazokomponenten Farbstoffe, die Papier in ähnlichen Tönen anfärben wie mit obiger Kupplungskomponente 1.7.

**Patentansprüche**

1. Triazinverbindungen, die in ihrer Betainform der Formel

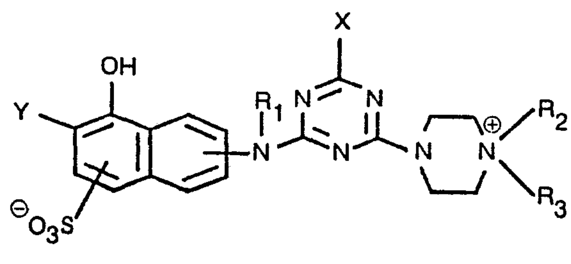

entsprechen, worin

$R_1$ Wasserstoff oder Methyl,

$R_2$ und $R_3$ unabhängig voneinander Wasserstoff, $C_1$- bis $C_4$-Alkyl, $C_3$- oder $C_4$-Alkenyl, Benzyl, Phenylethyl, die durch Hydroxy, $C_1$- bis $C_4$-Alkoxy, Halogen, Cyan, Amino oder Mono- und Di-$C_1$—$C_4$-alkylamino

oder deren Ammoniumverbindungen oder Tri-$C_1$—$C_4$-alkylammonium und der Benzyl- und Phenylethylrest zusätzlich durch $C_1$- bis $C_4$-Alkyl substituiert sein können,

X Halogen, Hydroxy, $C_1$—$C_4$-Alkoxy, $C_1$—$C_4$-Alkyl, Phenyl oder einen Rest der Formel

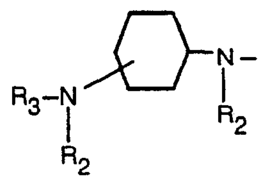

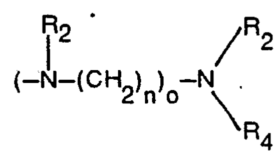

oder

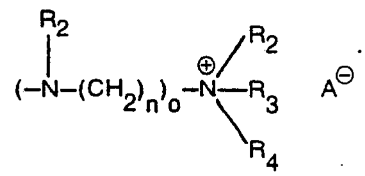

n 2 oder 3 und zusätzlich 0, wenn o = 1 ist,

o 0, 1 oder 2,

$A^{\ominus}$ ein Anion,

$R_4$ den Rest $R_2$ und zusätzlich gegebenenfalls durch Halogen, $C_1$—$C_4$-Alkyl, Hydroxy, $C_1$—$C_4$-Alkoxy und/oder den Rest

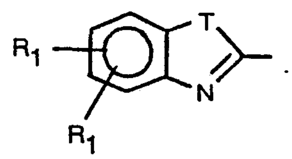

T = S, NH

substituiertes Phenyl oder Naphthyl, Benzthiazolyl (2) oder Isobenzthiazolyl (3) oder

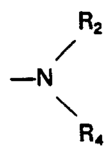

einen Pyrrolidin-, Piperidin- oder Morpholinring oder einen Ring der Formel

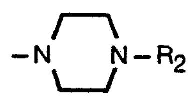

oder

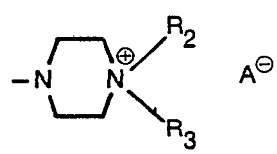

Y Wasserstoff oder den Rest D—(N=N—A)$_z$—N=N—,

D den Rest einer Diazokomponente der Benzol-, Naphthalin- oder heterocyclischen Reihe,

A den Rest einer Kupplungskomponente der Benzol- oder Naphthalinreihe und

m und z 0 oder 1 bedeuten, und worin

die Summe der basischen und kationischen Gruppen größer ist als die Anzahl der Sulfonsäuregruppen.

2. Triazinverbindungen der Formel des Anspruchs 1, in der Y für Wasserstoff und die Sulfonsäuregruppe in 3-Stellung stehen.

3. Triazinverbindungen gemäß Anspruch 1 der Formel

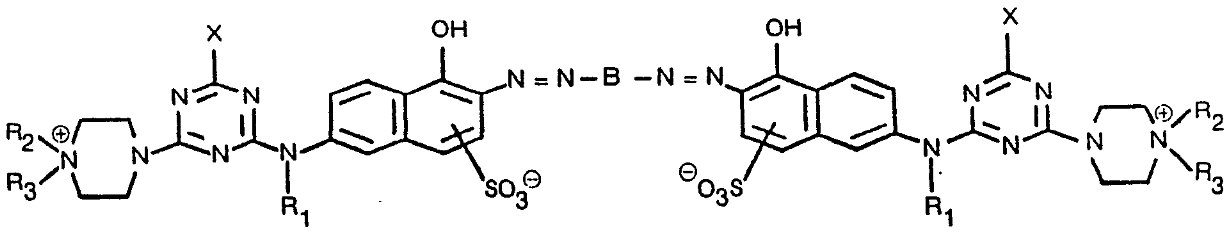

worin

B den Rest einer aromatischen Tetrazokomponente darstellt,

X, $R_1$, $R_2$ und $R_3$ die Bedeutung des Anspruchs 1 haben, und worin die Summe der basischen und kationischen Gruppen größer ist als die Anzahl der Sulfonsäuregruppen.

4. Triazinverbindungen gemäß Anspruch 1 der Formel

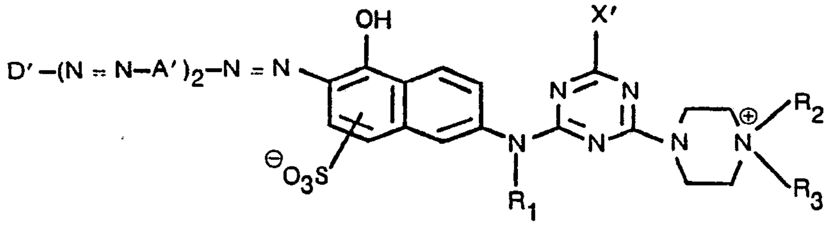

worin

X' einen Rest der Formel

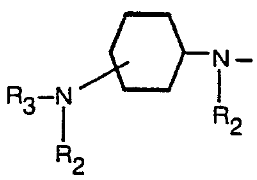

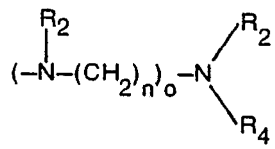

oder

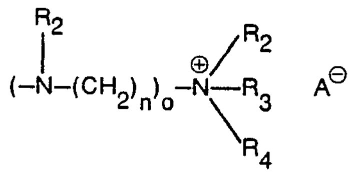

D' einen Phenyl-, Naphthyl-, Benzthiazolyl- oder Dibenzofuranylrest und

A' einen Phenylen- oder Naphthylenrest, bedeuten, wobei

D' und A' durch Halogen, $C_1$—$C_4$-Alkyl, $C_1$—$C_4$-Alkoxy, Acetylamino, Benzoylamino, Phenoxy, die Gruppen

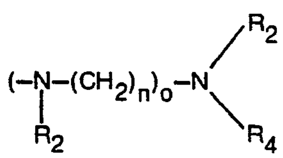

,

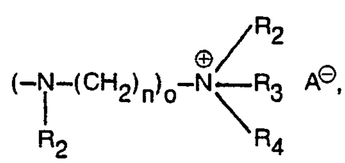

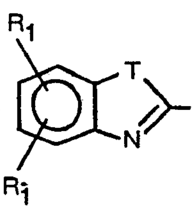

,

T = S, NH

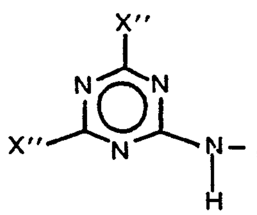

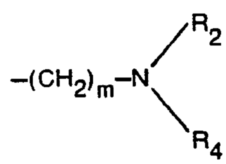

,

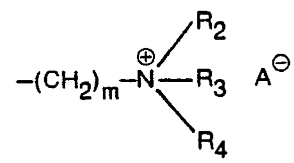

und/oder die Sulfonsäuregruppe substituiert sein können,

X'' den Rest X' und zusätzlich die Reste

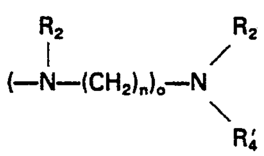

oder

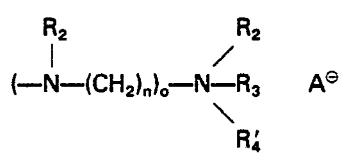

bedeutet, worin

$R_4'$ gegebenenfalls durch die Reste

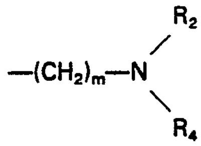

oder

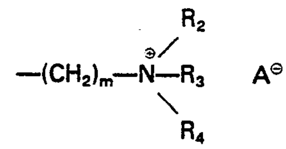

substituiertes Phenyl oder Naphthyl bedeutet, worin die übrigen Symbole die Bedeutung von Anspruch 1 haben, und die Summe der basischen und kationischen Gruppen größer ist als die Anzahl der Sulfonsäuregruppen.

5. Triazinverbindungen gemäß Anspruch 1 der Formel

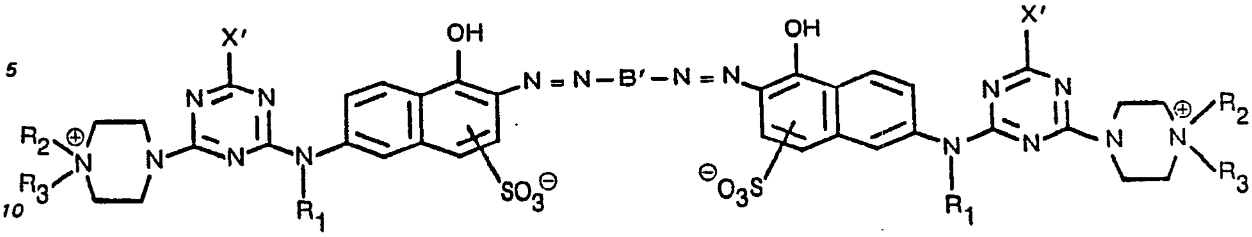

worin

B' einen Phenylen- oder Naphthylen-Rest oder einen Rest der Formel

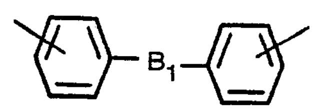

worin

$B_1$ eine direkte Bindung, $-(CH_2)_p-$, $-NH-CO-$, $-O-$, $-SO_2-$, $-NH-CO-NH-$, $-O(CH_2)_p-O-$, $-NH-CO-(CH_2-)_pCO-NH-$, $-CO-NH-(CH_2)_p-NHCO-$ oder

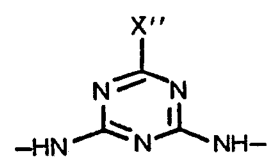

und

p 2 oder 3 bedeuten,

wobei die isocyclischen Ringe durch Halogen, $C_1-C_4$-Alkyl, $C_1-C_4$-Alkoxy, Acetylamino, Benzoylamino, Phenoxy, die Gruppen

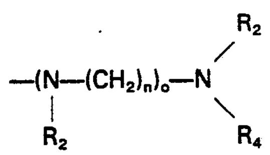
,
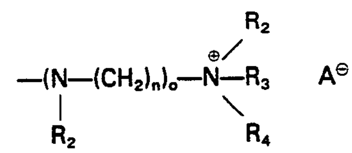

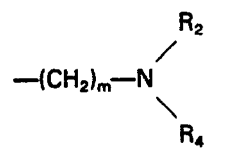
oder
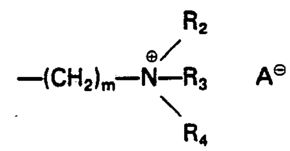

und/oder die Sulfonsäuregruppe substituiert sein können, worin die übrigen Symbole die in den Ansprüchen 1 und 4 angegebene Bedeutung haben und die Summe der basischen und kationischen Gruppen größer ist als die Anzahl der Sulfonsäuregruppen.

6. Verfahren zur Herstellung von Triazinverbindungen des Anspruchs 1 ($Y=D-(N=N-A)_z-N=N-$), dadurch gekennzeichnet, daß man Verbindungen der Formel

$$D-(N=N-A)_z-NH_2$$

diazotiert und mit Verbindungen der Formel

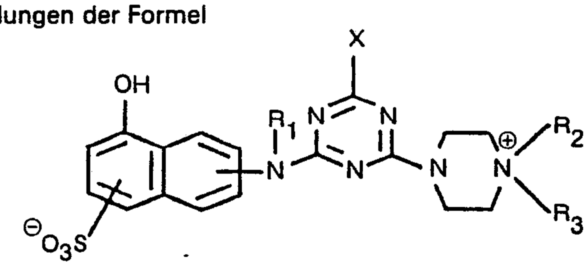

worin die Symbole die in Anspruch 1 angegebene Bedeutung haben, kuppelt.

7. Verfahren zur Herstellung von Triazinverbindungen des Anspruchs 2, dadurch gekennzeichnet, daß man Cyanurhalogenide in beliebiger Reihenfolge mit Verbindungen der Formel

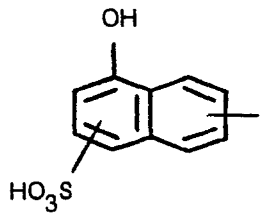 , XH , und 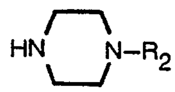 bzw. 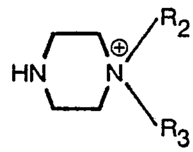 $A^{\ominus}$

worin die Symbole die in Anspruch 1 angegebene Bedeutung haben und $A^{\ominus}$ für ein Anion steht, umsetzt.

8. Verfahren zum Färben von natürlichen und synthetischen kationisch anfärbbaren Substraten und Massen, dadurch gekennzeichnet, daß man Farbstoffe des Anspruchs 1 $(Y{=}D{-}(N{=}N{-}A)_z{-}N{=}N{-})$ verwendet.

9. Verfahren zum Färben von Papier in der Masse und in der Oberfläche, dadurch gekennzeichnet, daß man Farbstoffe des Anspruchs 1 $(Y{=}D{-}(N{=}N{-}A)_z{-}N{=}N{-})$ verwendet.

10. Verwendung von Triazinverbindungen des Anspruchs 2 als Kupplungskomponenten.

**Revendications**

1. Composés triaziniques qui, sous leur forme bétaïne, répondent à la formule

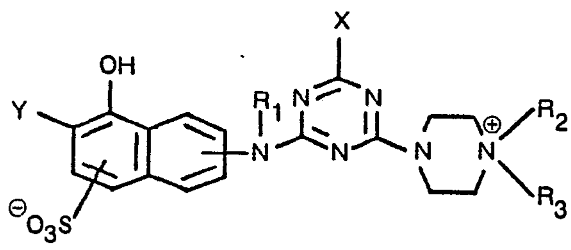

dans laquelle

$R_1$ représente l'hydrogène ou le groupe méthyle,

$R_2$ et $R_3$ représentent, indépendamment l'un de l'autre, l'hydrogène, un groupe alkyle en $C_1$ à $C_4$, alcényle en $C_3$ ou $C_4$, benzyle, phényléthyle, qui peuvent être substitués par un radical hydroxy, alkoxy en $C_1$ à $C_4$, halogéno, cyano, amino ou mono- et di(alkyle en $C_1$ à $C_4$)amino ou leurs composés d'ammonium ou tri-(alkyle en $C_1$ à $C_4$)ammonium et le reste benzyle et le reste phényléthyle peuvent en outre être substitués par un radical alkyle en $C_1$ à $C_4$,

X est un halogène, un groupe hydroxy, alkoxy en $C_1$ à $C_4$, alkyle en $C_1$ à $C_4$, phényle ou un reste de formule

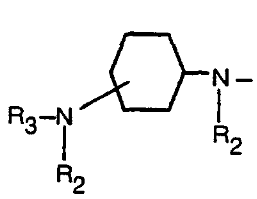 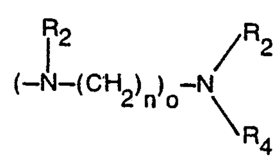 ou 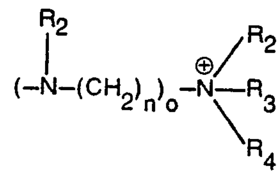 $A^{\ominus}$

n a la valeur 2 ou 3 et, en outre, la valeur 0 lorsque o est égal à 1,

o est égal à 0, 1 ou 2,

$A^{\ominus}$ désigne un anion,

$R_4$ représente le reste $R_2$ et en outre un groupe phényle ou naphtyle éventuellement substitué en outre par un halogène, un radical alkyle en $C_1$ à $C_4$, hydroxy, alkoxy en $C_1$ à $C_4$ et/ou le reste

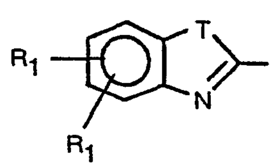 T = S, NH

le groupe benzothiazolyle (2) ou isobenzothiazolyle (3), ou

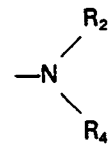

représente un noyau de pyrrolidine, pipéridine ou morpholine ou un noyau de formule

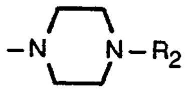 ou 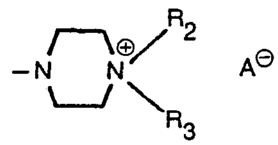

Y représente l'hydrogène ou le reste $D—(N=N—A)_z—N=N—$,
D est le reste d'un composant diazotable de la série benzénique, naphtalénique ou hétérocyclique,
A est le reste d'un copulant de la série du benzène ou du naphtalène et
m et z ont la valeur 0 ou 1, et
la somme des groupes basiques et cationiques est supérieure au nombre des groupes acide sulfonique.

2. Composés triaziniques de formule suivant la revendication 1, dans laquelle Y représente l'hydrogène et le groupe acide sulfonique est en position 3.

3. Composés triaziniques suivant la revendication 1, de formule

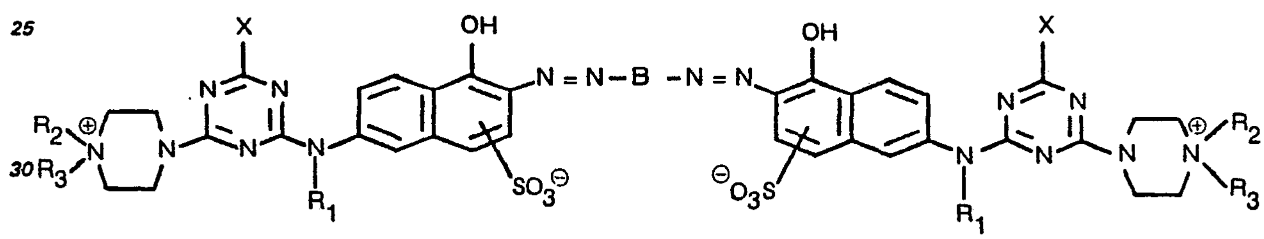

dans laquelle
B est le reste d'un composant tétrazoïque aromatique,
X, $R_1$, $R_2$ et $R_3$ ont la définition donnée dans la revendication 1, et la somme des groupes basiques et cationiques est supérieure au nombre des groupes acide sulfonique.

4. Composés triaziniques suivant la revendication 1, de formule

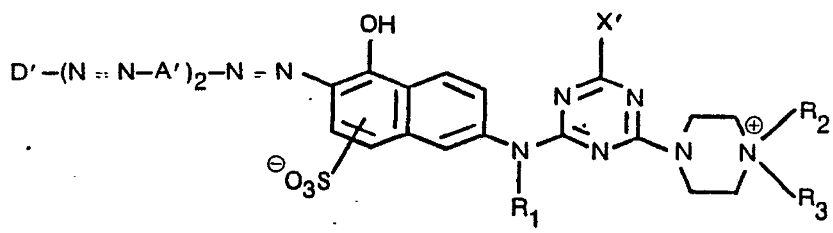

dans laquelle
X' est un reste de formule

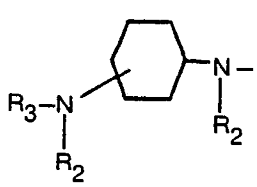 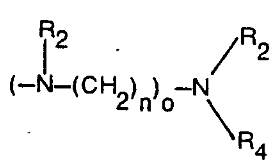 ou 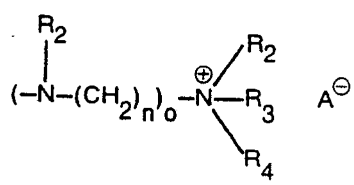

D' est un reste phényle, naphtyle, benzothiazolyle ou dibenzofurannyle et
A' est un reste phénylène ou naphtylène,

D′ et A′ peuvent être substitués par un radical halogéno, alkyle en $C_1$ à $C_4$, alkoxy en $C_1$ à $C_4$, acétyl-amino, benzoylamino, phénoxy, les groupes

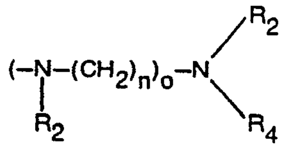 , 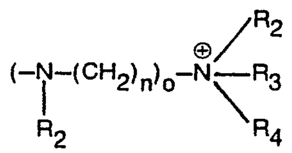 A⊖, 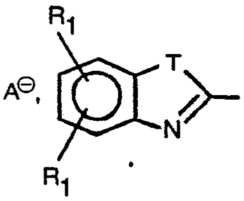 ,

T = S, NH

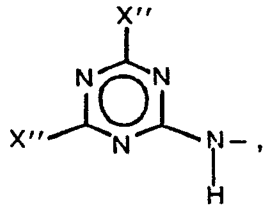 , 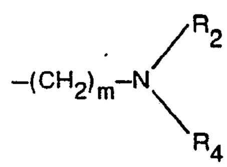 , 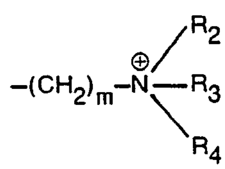 A⊖

et/ou le groupe acide sulfonique,

X″ représente le reste X′ et, en outre, les restes

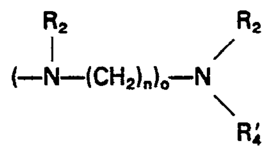 ou 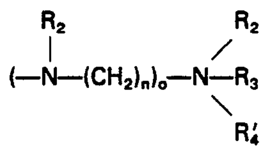 A⊖ .

où

R′₄ désigne le groupe phényle ou naphtyle éventuellement substitué par les restes

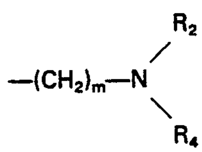 ou 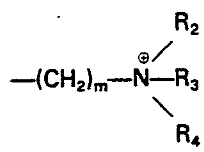 A⊖

où les autres symboles ont la définition donnée dans la revendication 1 et la somme des groupes basiques et cationiques est supérieure au nombre des groupes acide sulfonique.

5. Composés triaziniques suivant la revendication 1, de formule

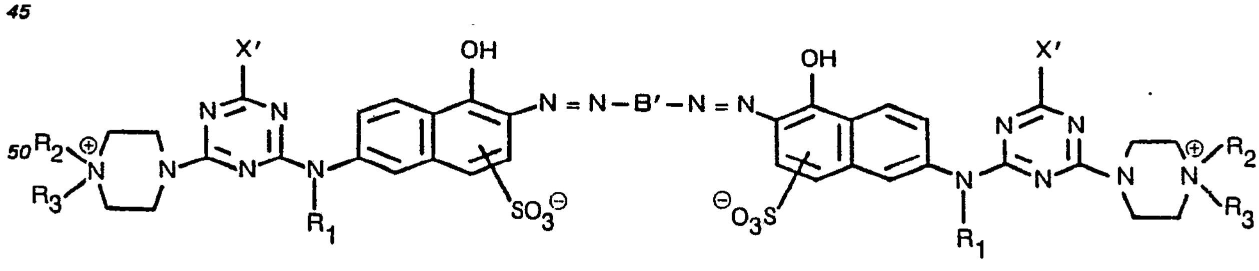

dans laquelle

B′ est un reste phénylène ou naphtylène ou un reste de formule

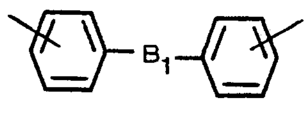

dans laquelle

$B_1$ est une liaison directe, $-(CH_2)_p-$, $-NH-CO$; $-O-$, $-SO_2-$, $-NH-CO-NH-$, $-O(CH_2)_p-O-$, $-NH-CO-(CH_2-)_pCO-NH-$, $-CO-NH-(CH_2)_p-NHCO-$ ou

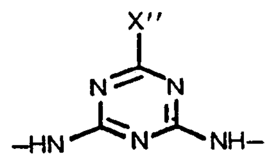

et

p a la valeur 2 ou 3,

les noyaux isocycliques pouvant être substitués par un radical halogéno, alkyle en $C_1$ à $C_4$, alkoxy en $C_1$ à $C_4$, acétylamino, benzoylamino, phénoxy, les groupes

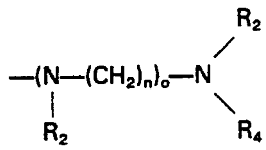 , 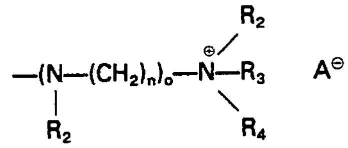

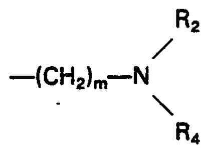 ou 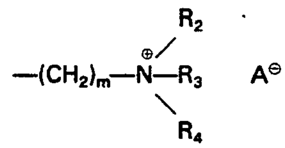

et/ou le groupe acide sulfonique, les autres symboles ayant la définition indiquée dans les revendications 1 à 4 et la somme des groupes basiques et cationiques étant supérieure au nombre de groupes acide sulfonique.

6. Procédé de production de composés triaziniques suivant la revendication 1 ($Y=D-(N=N-A)_z-N=N-$), caractérisé en ce qu'on diazote des composés de formule

$$D-(N=N-A)_z-NH_2$$

et on les fait copuler avec des composés de formule

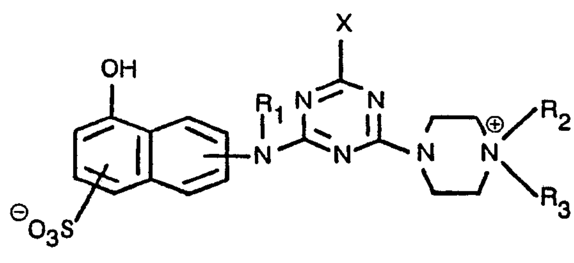

dans laquelle les symboles ont la définition indiquée dans la revendication 1.

7. Procédé de production de composés triaziniques suivant la revendication 2, caractérisé en ce qu'on fait réagir des halogénures de cyanuryle dans un ordre quelconque avec des composés de formules

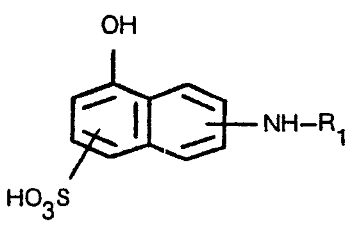 , XH , et 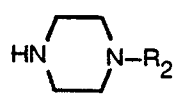 ou 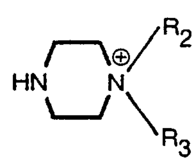 $A^{\ominus}$

dans lesquelles les symboles ont la définition indiquée dans la revendication 1 et $A^{\ominus}$ représente un anion.

8. Procédé de teinture de substrats et de mélanges naturels et synthétiques susceptibles de teinture cationique, caractérisé en ce qu'on utilise des colorants suivant la revendication 1 ($Y=D-(N=N-A)_z-N=N-$).

9. Procédé de coloration de papier dans la masse et en surface, caractérisé en ce qu'on utilise des colorants suivant la revendication 1 ($Y=D-(N=N-A)_z-N=N-$).

10. Utilisation de composés triaziniques suivant la revendication 2, comme copulants.

**Claims**

1. Triazine compounds which, in their betaine form, correspond to the formula

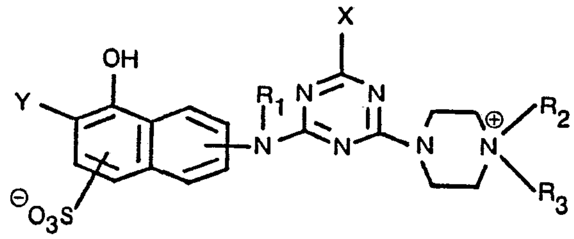

wherein

$R_1$ denotes hydrogen or methyl,

$R_2$ and $R_3$ independently of one another denote hydrogen, $C_1$- to $C_4$-alkyl, $C_3$- or $C_4$-alkenyl, benzyl or phenylethyl, which can be substituted by hydroxyl, $C_1$- to $C_4$-alkoxy, halogen, cyano, amino or mono- and di-$C_1$—$C_4$-alkylamino or ammonium compounds thereof or tri-$C_1$—$C_4$-alkyl-ammonium and the benzyl radical and phenylethyl radical additionally by $C_1$- to $C_4$-alkyl,

X denotes halogen, hydroxyl, $C_1$—$C_4$-alkoxy, $C_1$—$C_4$-alkyl, phenyl or a radical of the formula

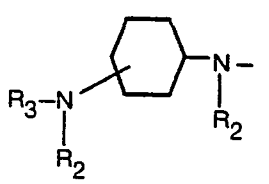
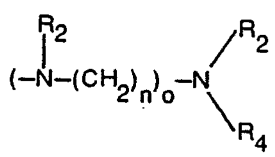
or
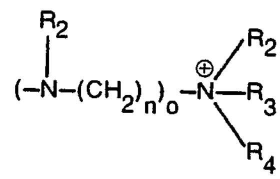
$A^{\ominus}$

n denotes 2 or 3 and additionally 0 if o = 1,

o denotes 0, 1 or 2,

$A^{\ominus}$ denotes an anion,

$R_4$ denotes the radical $R_2$ and additionally naphthyl or phenyl optionally substituted by halogen, $C_1$—$C_4$-alkyl, hydroxyl, $C_1$—$C_4$-alkoxy and/or the radical

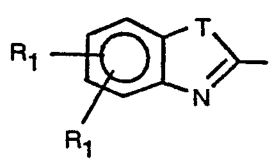
T = S, NH

benzthiazol-2-yl or isobenzthiazol-3-yl, or

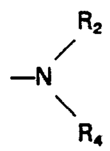

denotes a pyrrolidine, piperidine or morpholine ring or a ring of the formula

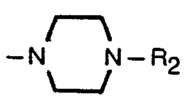
or
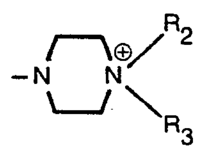
$A^{\ominus}$

Y denotes hydrogen or the radical D—(N=N—A)$_z$—N=N—,

D denotes the radical of a diazo component of the benzene, naphthalene or heterocyclic series,

A denotes the radical of a coupling component of the benzene or naphthalene series and

m and z denote 0 or 1, and wherein

the sum of the basic and cationic groups is greater than the number of sulphonic acid groups.

2. Triazine compounds of the formula of Claim 1, in which Y represents hydrogen and the sulphonic acid group is in the 3-position.

3. Triazine compounds according to Claim 1 of the formula

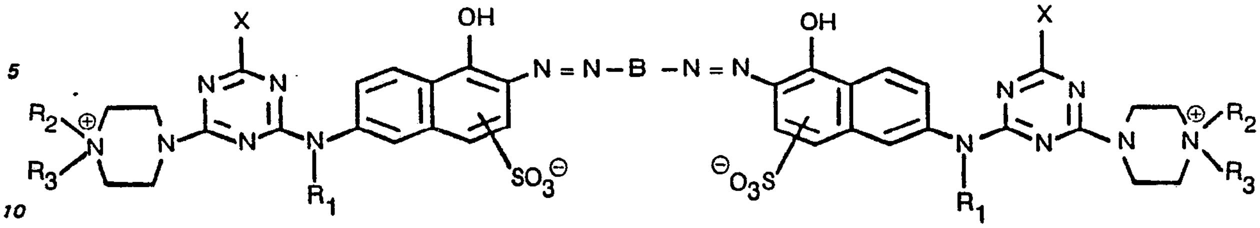

wherein

B represents the radical of an aromatic tetrazo component,

X, $R_1$, $R_2$ and $R_3$ have the meaning of Claim 1, and wherein the sum of the basic and cationic groups is greater than the number of sulphonic acid groups.

4. Triazine compounds according to Claim 1 of the formula

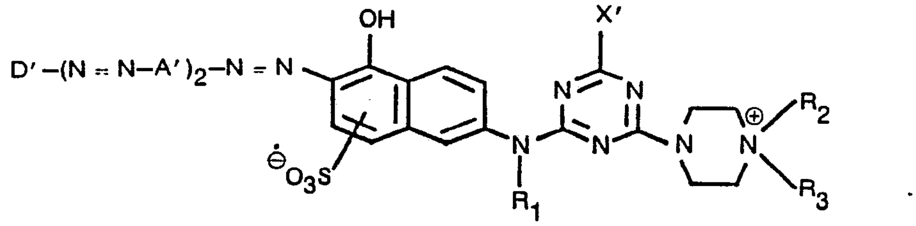

wherein

X' denotes a radical of the formula

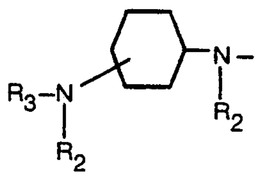 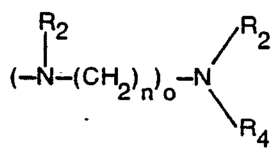 or 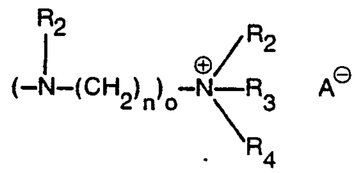

D' denotes a phenyl, naphthyl, benzthiazolyl or dibenzofuranyl radical and

A' denotes a phenylene or naphthylene radical,

it being possible for D' and A' to be substituted by halogen, $C_1$—$C_4$-alkyl, $C_1$—$C_4$-alkoxy, acetylamino, benzoylamino, phenoxy, the groups

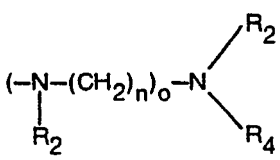 , 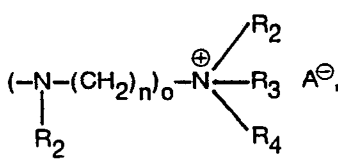 , 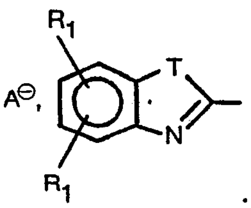 ,

T = S, NH

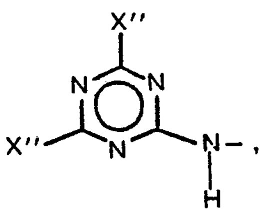 , 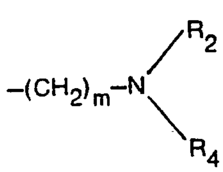 , 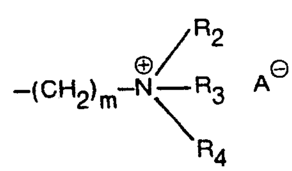

and/or the sulphonic acid group,

X'' denotes the radical X' and additionally the radicals

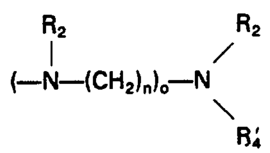 or 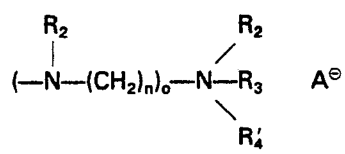

wherein

$R_4'$ denotes naphthyl or phenyl optionally substituted by the radicals

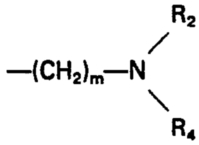

or

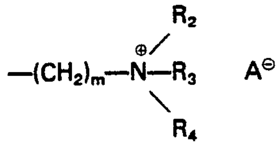

the remaining symbols having the meaning of Claim 1, and the sum of the basic and cationic groups being greater than the number of sulphonic acid groups.

5. Triazine compounds according to Claim 1 of the formula

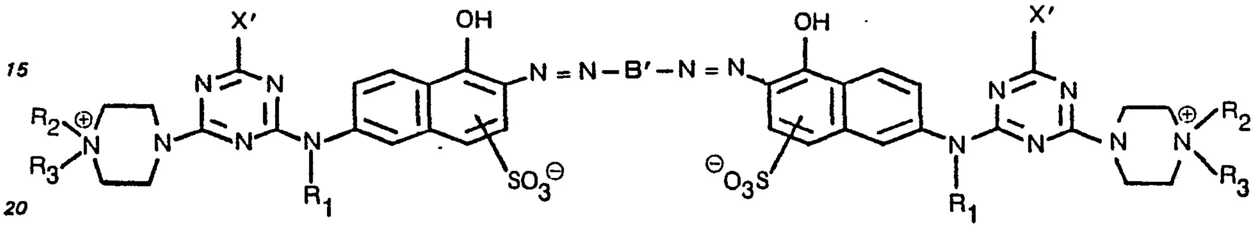

wherein

B′ denotes a phenylene or naphthylene radical or a radical of the formula

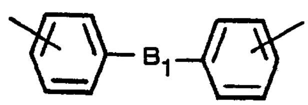

wherein

$B_1$ denotes a direct bond, $—(CH_2)_p—$, —NH—CO—, —O—, $—SO_2—$, —NH—CO—NH—, $—O(CH_2)_p—O—$, $—NH—CO—(CH_2—)_pCO—NH—$, $—CO—NH—(CH_2)_p—NHCO—$ or

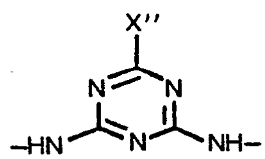

and

p denotes 2 or 3,

it being possible for the isocyclic rings to be substituted by halogen, $C_1$—$C_4$-alkyl, $C_1$—$C_4$-alkoxy, acetylamino, benzoylamino, phenoxy, the groups

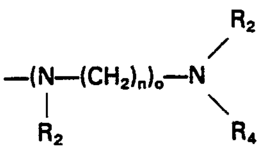

,

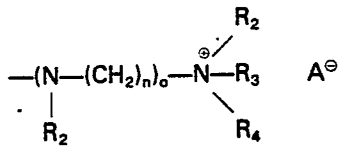

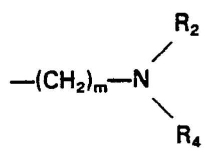

or

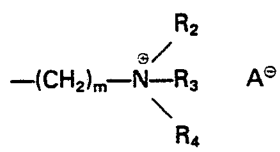

and/or the sulphonic acid group, the remaining symbols having the meaning given in Claims 1 and 4 and the sum of the basic and cationic groups being greater than the number of sulphonic acid groups.

6. Process for the preparation of triazine compounds of Claim 1 $(Y=D—(N=N—A)_z—N=N—)$, characterised in that compounds of the formula

$$D—(N=N—A)_z—NH_2$$

are diazotised and coupled with compounds of the formula

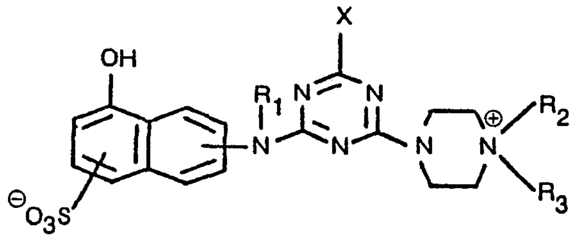

wherein the symbols have the meaning given in Claim 1.

7. Process for the preparation of triazine compounds of Claim 2, characterised in that cyanuric halides are reacted, in any desired sequence, with compounds of the formula

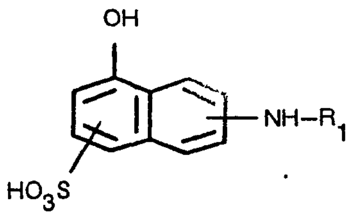 , XH , und 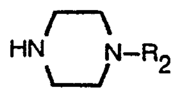 bzw. 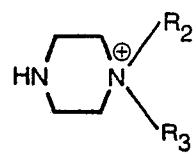 $A^{\ominus}$

wherein the symbols have the meaning given in Claim 1 and $A^{\ominus}$ represents an anion.

8. Process for dyeing natural and synthetic cationically dyeable substrates and compositions, characterised in that dyestuffs of Claim 1 ($Y=D-(N=N-A)_z-N=N-$) are used.

9. Process for beater-dyeing and surface-dyeing paper, characterised in that dyestuffs of Claim 1 ($Y=D-(N=N-A)_z-N=N-$) are used.

10. Use of triazine compounds of Claim 2 as coupling components.